# EUROPEAN PATENT APPLICATION

(11) **EP 1 930 435 A1**
(43) Date of publication of application: **11.06.2008**
(21) Application number: 06810701.0
(22) Date of filing: 28.09.2006
(51) Int. Cl.: C12N 15/09, C12P 1/04

(54) **PROCESS FOR PRODUCING USEFUL SUBSTANCE**

(30) Priority: 29.09.2005 JP 2005284539
(71) Applicant: KYOWA HAKKO KOGYO CO., LTD., Chiyoda-ku, Tokyo 100-8185 (JP)
(72) Inventor: HIBI, Makoto, (JP); MORI, Hideo, (JP)
(74) Representative: Bohmann, Armin K.
(86) International application number: PCT/JP2006/319243
(87) International publication number: WO 2007/037300

(57) **Abstract**

The present invention provides a process for producing a useful substance by use of a microorganism that lacks in their chromosomal DNA all or part of a gene encoding a protein having the amino acid sequence shown in SEQ ID NO: 1 or a gene encoding a protein having 80% or more, preferably 90% or more, more preferably 95% or more, even more preferably 97% or more, still more preferably 98% or more, and yet more preferably 99% or more homology with the amino acid sequence shown in SEQ ID NO: 1.

## Description

### Technical Field

The present invention relates to a process for producing a useful substance using a microorganism.

### Background Art

Many processes for producing useful substances, such as amino acids, using microorganisms have been reported previously. Most of them use: (1) microorganisms in which the expression of a biosynthetic gene for a useful substance has been enhanced; (2) microorganisms having a desensitized mutation in such a gene; (3) microorganisms in which a gene encoding an enzyme that degrades a useful substance has been disrupted; or the like (Non-Patent Documents 1 to 3).

Known processes for producing useful substances using microorganisms, other than those described above in (1) to (3), include processes for producing lysine using *E. coli* in which the *rmf* gene encoding a protein having an activity to reduce the growth rate has been disrupted (Patent Document 1). Generally, however, it is difficult to identify genes that are indirectly involved in the enhancement of biosynthesis or the suppression of degradation of useful substances.

The entire nucleotide sequences of the chromosomal DNA for many microorganisms have been determined (Non-Patent Document 4). Furthermore, methods for deleting a specific gene or a region from the chromosomal DNA of a microorganism, according to one's intentions, by using homologous recombination techniques are also known (Non-Patent Document 5). In addition, a library of one-gene-disruption mutants of almost all genes in a microorganism's chromosomal DNA, libraries of mutant strains in which deletable regions of about 20 kbp in the chromosomal DNA have been thoroughly and separately deleted, and the like have been prepared using the information of the entire nucleotide sequence of chromosomal DNA and homologous recombination techniques (Non-Patent Documents 6 and 7).

The *yhbC* gene is an *E. coli* gene having unknown function, though its nucleotide sequence is already known (Non-Patent Document 8). There are reports describing the *yhbC* gene product as essential for the growth of *E. coli* (Patent Documents 2 and 3). Meanwhile, a mutant strain has been produced, in which the *yhbC* gene in the chromosomal DNA of *E. coli* has been disrupted. This strain is available from the Nara Institute of Science and Technology (Non-Patent Documents 9 and 10).

However, it is not presently known that productivity of a useful substance may be improved in *E. coli* that lack all or part of the *yhbC* gene from the chromosomal DNA.
[Patent Document 1] Japanese Patent Application Kokai Publication No. (JP-A) 2002-306191 (unexamined, published Japanese patent application)
[Patent Document 2] Japanese Patent Kohyo Publication No. (JP-A) 2002-541819 (unexamined Japanese national phase publication corresponding to a non-Japanese international publication)
[Patent Document 3] JP-A (Kohyo) 2002-541820
[Non-Patent Document 1] Microbial Production of L-Amino Acids, ADVANCES IN BIOCHEMICAL ENGINEERING BIOTECHNOLOGY, vol. 79 (2003), Springer
[Non-Patent Document 2] Kakusan Hakkou (Nucleic acid fermentation) Kodansha Scientific, 1976
[Non-Patent Document 3] J. Biosci. Bioeng. (Journal of Bioscience and Bioengineering), 90, 522 (2000)
[Non-Patent Document 4] http://www.tigr.org/tdb/mdb/mdbcomplete.html
[Non-Patent Document 5] J. Bacteriol. (Journal of Bacteriology), 180, 2063 (1998)
[Non-Patent Document 6] Tanpakushitsu Kakusan Koso (Protein, Nucleic acid, and Enzyme), Vol. 46, p. 2386 (2001)
[Non-Patent Document 7] Nature Biotechnol. (Nature Biotechnology), 20, 1018 (2002) [Non-Patent Document 8] Science, 277, 1453 (1997)
[Non-Patent Document 9] http://ecoli.aist-nara.ac.jp/GB5/search.jsp
[Non-Patent Document 10] http://www.shigen.nig.ac.jp/ecoli/strain/deletionQueryAction.do

### Disclosure of the Invention

### [Problems to be Solved by the Invention]

An objective of the present invention is to provide a process for producing a useful substance using a microorganism having improved productivity.

### [Means for Solving the Problems]

The present invention relates to following (1) to (6):
(1) a process for producing a useful substance, which comprises culturing a microorganism that lacks from its chromosomal DNA all or part of a gene encoding a protein comprising the amino acid sequence of SEQ ID NO: 1 or a gene encoding a protein that has 80% or more homology to the amino acid sequence of SEQ ID NO: 1 in a medium so as to produce and accumulate the useful substance in the culture, and recovering the useful substance from the culture;
(2) the process according to (1), wherein the gene encoding a protein comprising the amino acid sequence of SEQ ID NO: 1 has the nucleotide sequence of SEQ ID NO: 2;
(3) the process according to (1) or (2), wherein the ability of the microorganism to produce the useful substance has been improved by a method selected from the following [1] to [5]:
   [1] a method of partially or completely releasing at least one of the mechanisms that regulate the biosynthesis of the useful substance;
   [2] a method of enhancing expression of at least one of the enzymes involved in the biosynthesis of the useful substance;
   [3] a method of increasing the copy number of at least one of the enzyme genes involved in the biosynthesis of the useful substance;
   [4] a method of partially or completely blocking at least one of the branched metabolic pathways of the biosynthetic pathway of the useful substance to metabolites other than the useful substance; and
   [5] a method of selecting a cell strain that is more resistant to an analog of the useful substance as compared with a wild-type strain;
(4) the process according to any one of (1) to (3), wherein the microorganism belongs to the genus *Escherichia, Erwinia, Pseudomonas, Rhodobacter, Salmonella, Serratia, Vibrio, Xanthomonas,* or *Xylella;*
(5) the process according to any one of (1) to (4), wherein the microorganism is a microorganism of the species *Escherichia coli, Erwinia carotovora, Pseudomonas putida, Pseudomonas fluorescens, Pseudomonas aeruginosa, Rhodobacter capsulatus, Salmonella typhimurium, Serratia marcescens, Vibrio fischeri, Xanthomonas capestris,* or *Xylella fastidiosa;* and
(6) the process according to any one of (1) to (5), wherein the useful substance is selected from the group consisting of a protein, peptide, amino acid, nucleic acid, vitamin, sugar, sugar alcohol, alcohol, organic acid, and lipid.

### [Effects of the Invention]

Useful substances can be efficiently produced according to the present invention.

### Best Mode for Carrying out the Invention

### 1. Microorganisms used in the production process of the present invention

Microorganisms used in the production process of the present invention include microorganisms that lack all or part of a gene encoding a protein comprising the amino acid sequence shown in SEQ ID NO: 1 (hereinafter sometimes referred to as the YhbC protein) or a gene encoding a protein having 80% or higher, preferably 90% or higher, more preferably 95% or higher, even more preferably 97% or higher, still more preferably 98% or higher, and most preferably 99% or higher homology to the amino acid sequence shown in SEQ ID NO: 1 (hereinafter sometimes referred to as an YhbC protein homologue). A "gene" described herein includes a structural gene, and regions having particular regulatory function, such as promoter and operator.

Homology of amino acid and nucleotide sequences can be determined using the algorithm BLAST by Karlin and Altschul [Pro. Natl. Acad. Sci. USA, 90, 5873 (1993)] and the algorithm FASTA [Methods Enzymol., 183, 63 (1990)]. Programs called BLASTN and BLASTX have been developed based on this algorithm, BLAST [J. Mol. Biol., 215, 403 (1990)]. When nucleotide sequences are analyzed by BLASTN based on BLAST, the parameters may be set, for example, as score = 100 and wordlength =12. When amino acid sequences are analyzed by BLASTX based on BLAST, the parameters may be set, for example, as score = 50 and wordlength = 3. When BLAST and Gapped BLAST programs are used, default parameters are used for each program. Specific procedures for these analytical methods are well known (http://www.ncbi.nlm.nih.gov.).

Microorganisms that lack all or part of a gene encoding a YhbC protein or a gene encoding a YhbC protein homologue (hereinafter sometimes referred to as the *yhbC* gene or homologue gene thereof) include:
(1) microorganisms that do not express the YhbC protein or a homologue protein thereof as a result of failure of transcriptional regulation, for example by a promoter or an operator of a *yhbC* gene or a homologue gene thereof, due to the deletion of nucleotide(s) from the nucleotide sequence of the gene in the microorganism's chromosomal DNA;
(2) microorganisms that do not express the YhbC protein or a homologue protein thereof as an active protein as a result of a frameshift occurring within the structural gene of the *yhbC* gene or a homologue gene thereof due to the deletion of nucleotide(s) from the nucleotide sequence of the gene in the microorganism's chromosomal DNA; and
(3) microorganisms that do not express an active protein due to the fact that they lack all or part of a structural gene from the *yhbC* gene or a homologue gene thereof as a result of the deletion of nucleotide(s) from the nucleotide sequence of the gene in the microorganism's chromosomal DNA. Preferred microorganisms are those of (3).

More specifically, the microorganisms described above in (3) include microorganisms that do not express an active protein because all or part of a structural gene in the nucleotide sequence of SEQ ID NO: 2 is deleted. A partial deletion includes a single nucleotide deletion within the structural gene in the nucleotide sequence of SEQ ID NO: 2, so long as the deletion results in the loss of an activity of the protein comprising the amino acid sequence of SEQ ID NO: 1. Preferred deletions are those of 5 to 100 nucleotides within the structural gene, more preferably those of 10 to 100 nucleotides within the gene, and even more preferably those of 50 to 100 nucleotides within the gene.

In the context of the present invention, a useful substance produced by microorganisms used in the production process herein may be any substance of interest, so long as it is an industrially useful substance that can be produced by microorganisms. Such substances preferably include proteins, peptides, amino acids, nucleic acids, vitamins, sugars, sugar alcohols, alcohols, organic acids, and lipids. More preferably, such proteins include inosine kinase, glutamate 5-kinase (EC 2.7.2.11), glutamate-5-semialdehyde dehydrogenase (EC 1.2.1.41), pyrroline-5-carboxylate reductase (EC 1.5.1.2), xylose reductase, P450, and human granulocyte colony-stimulating factor; peptides include glutathione; amino acids include L-alanine, glycine, L-glutamine, L-glutamic acid, L-asparagine, L-aspartic acid, L-lysine, L-methionine, L-threonine, L-leucine, L-valine, L-isoleucine, L-proline, L-histidine, L-arginine, L-tyrosine, L-tryptophan, L-phenylalanine, L-serine, L-cysteine, L-3-hydroxyproline, and L-4-hydroxyproline; nucleic acids include inosine, guanosine, inosinic acid, and guanylic acid; vitamins include riboflavin, thiamine, and ascorbic acid; sugars include xylose; sugar alcohols include xylitol; alcohols include ethanol; organic acids include lactic acid and succinic acid; and lipids include eicosapentaenoic acid (EPA) and docosahexaenoic acid (DHA).

Microorganisms to be used in the production process of the present invention may belong to any genus, so long as their chromosomal DNA lacks all or part of the *yhbC* gene or a homologue gene thereof as described above. Such microorganisms preferably include prokaryotes, and more preferably bacteria.

Examples of such bacteria include microorganisms belonging to the genus *Escherichia, Erwinia, Pseudomonas, Rhodobacter, Salmonella, Serratia, Vibrio, Xanthomonas,* or *Xylella,* preferably *Escherichia coli, Erwinia carotovora, Pseudomonas putida, Pseudomonas fluorescens, Pseudomonas aeruginosa, Rhodobacter capsulatus, Salmonella typhimurium, Serratia marcescens, Vibrio fischeri, Xanthomonas capestris,* and *Xylella fastidiosa,* and more preferably *E. coli.*

### 2. Preparation of microorganisms useful in the production process of the present invention:

Microorganisms useful in the production process of the present invention can be prepared by:
(1) deleting all or part of an *yhbC* gene or a homologue gene thereof from the chromosomal DNA of a microorganism having the ability to produce a useful substance; or
(2) conferring the ability to produce useful substances to a microorganism that lacks all or part of an *yhbC* gene or a homologue gene thereof in its chromosomal DNA.

Microorganisms having the ability to produce useful substances include any microorganism, so long as they have the ability to produce one or more types of useful substances. Examples of such microorganisms include strains isolated from nature that themselves have the desired ability as well as microorganisms in which the ability to produce desired useful substances is artificially conferred by known methods. Such methods include:
(a) methods of partially or completely releasing at least one of the mechanisms that regulate the biosynthesis of useful substances;
(b) methods of potentiating the expression of at least one of the enzymes involved in the biosynthesis of useful substances;
(c) methods of increasing the copy number of at least one of the enzyme genes involved in the biosynthesis of useful substances;
(d) methods of partially or completely blocking at least one of the branched metabolic pathways of the biosynthetic pathway of a useful substance to metabolites other than the useful substance; and
(e) methods of selecting a cell strain that is more resistant to an analog of a useful substance as compared with a wild-type strain.

The known methods described above can be used alone or in combination.

Specifically, when the useful substance is an amino acid, for example, the methods listed above in (a) to (e) include: the example of method (a) described in Agric. Biol. Chem., 43, 105-111 (1979); J. Bacteriol., 110, 761-763 (1972); Appl. Microbiol. Biotechnol., 39, 318-323 (1993); and others; the example of method (b) described in Agric. Biol. Chem., 43, 105-111 (1979); J. Bacteriol., 110, 761-763 (1972); and others; the example of method (c) described in Appl. Microbiol. Biotechnol., 39, 318-323 (1993); Agric. Biol. Chem., 39, 371-377 (1987); and others; the example of method (d) described in Appl. Environ. Microbiol., 38, 181-190 (1979); Agric. Biol. Chem., 42, 1773-1778 (1978); and others; the example of method of (e) described in Agric. Biol. Chem., 36, 1675-1684 (1972); Agric. Biol. Chem., 41, 109-116 (1977); Agric. Biol. Chem., 37, 2013-2023 (1973); Agric. Biol. Chem., 51, 2089-2094 (1987); and others. Microorganisms having the ability to produce and accumulate various amino acids can be prepared according to the protocols described in the documents listed above and others.

Many illustrative examples of methods for preparing microorganisms having the ability to produce and accumulate amino acids, according to an above method of (a) to (e) or a combination thereof, are described in Biotechnology 2nd Ed., Vol. 6, Products of Primary Metabolism (VCH Verlagsgesellschaft mbH, Weinheim, 1996) section 14a and 14b; Advances in Biochemical Engineering/Biotechnology 79, 1-35 (2003); and Aminosan Hakkou (Amino acid fermentation), Gakkai Shuppan Center (Japan Scientific Societies Press), Hiroshi Aida et al., (1986). In addition to the above, there are many reports of methods for preparing microorganisms having the ability to produce and accumulate specific amino acids: JP-A (Kokai) 2003-164297; Agric. Biol. Chem., 39, 153-160 (1975); Agric. Biol. Chem., 39, 1149-1153 (1975); JP-A (Kokai) S58-13599; J. Gen. Appl. Microbiol., 4, 272-283 (1958); JP-A (Kokai) S63-94985; Agric. Biol. Chem., 37, 2013-2023 (1973); WO 97/15673; JP-A (Kokai) S56-18596; JP-A (Kokai) S56-144092; JP-A (Kohyo) 2003-511086; and others. Microorganisms having the ability to produce one or more types of amino acids can be prepared according to the protocols described in the documents listed above and others.

There are also many reports of methods for conferring the ability to produce useful substances other than amino acids to microorganisms. Any known method can be used to prepare microorganisms to be used in the production process of the present invention.

Methods of obtaining microorganisms that lack all or part of a *yhbC* gene or a homologue gene thereof in their chromosomal DNA are not limited, so long as the microorganisms can be obtained by such a method. For example, the microorganisms can be obtained by introducing nucleotide deletions into the *yhbC* gene or a homologue gene thereof in the chromosomal DNA of the microorganism, using nucleotide sequence information for a gene that encodes a protein comprising the amino acid sequence of SEQ ID NO: 1 and that is contained in the chromosomal DNA of the microorganisms listed below or a homologue gene of the *yhbC* gene.

The nucleotide sequence of a homologue gene of a *yhbC* gene can be determined by identifying and obtaining a homologue gene of the *yhbC* gene via Southern hybridization with the chromosomal DNA of various microorganisms, using as a probe all or part of a DNA having the nucleotide sequence complementary to the nucleotide sequence of SEQ ID NO: 2; or via PCR, using chromosomal DNAs of various microorganisms as templates and DNAs designed based on the nucleotide sequence of SEQ ID NO: 2 as primers; and then analyzing the nucleotide sequence of the gene according to a conventional method.

Chromosomal DNAs subjected to Southern hybridization or PCR may be derived from any microorganisms. Such chromosomal DNAs preferably include those of microorganisms belonging to the genus *Escherichia, Erwinia, Pseudomonas, Rhodobacter, Salmonella, Serratia, Vibrio, Xanthomonas,* or *Xylella,* more preferably those of *Escherichia coli, Erwinia carotovora, Pseudomonas putida, Pseudomonas fluorescens, Pseudomonas aeruginosa, Rhodobacter capsulatus, Salmonella typhimurium, Serratia marcescens, Vibrio fischeri, Xanthomonas capestris,* or *Xylella fastidiosa.*

The "hybridization" described above means that a DNA hybridizes to another DNA having a specific nucleotide sequence, or a portion thereof. Thus, a DNA having a specific nucleotide sequence or a portion thereof can be used as a probe in northern or Southern blot analysis, or as an oligonucleotide primer in PCR analysis. DNAs used as probes include DNAs of at least 100 nucleotides or more, preferably 200 nucleotides or more, and more preferably 500 nucleotides or more. DNAs used as primers include DNAs of at least 10 nucleotides or more, preferably 15 nucleotides or more.

Methods for DNA hybridization experiments are well known. For example, those skilled in the art can determine hybridization conditions according to the present specification. Examples of such hybridization conditions are described in Molecular Cloning, 2nd Ed., or 3rd Ed., (2001); Methods for General and Molecular Bacteriology, ASM Press (1994); Immunology methods manual, Academic press (Molecular); and others. Such methods can be conducted according to protocols described in many other standard textbooks as well.

Hybridization is preferably carried out under stringent conditions. A preferred stringent condition is: overnight incubation at 42°C of probe DNA and filter with immobilized DNA in a solution containing 50% formamide, 5x SSC (750 mM sodium chloride and 75 mM sodium citrate), 50 mM sodium phosphate (pH 7.6), 5x Denhardt's solution, 10% dextran sulfate, and 20 µg/l denatured salmon sperm DNA, followed by, for example, washing of the filter in a solution of 0.2x SSC at about 65°C. Lower stringency conditions may also be used. Stringent conditions can be altered by adjusting the formamide concentration (the stringency becomes lower as the formamide concentration is lowered), or by altering salt concentrations or temperature conditions. An example of a low stringency condition includes overnight incubation at 37°C in a solution containing 6x SSCE (20x SSCE contains 3 mol/l sodium chloride, 0.2 mol/l sodium dihydrogenphosphate, and 0.02 mol/l EDTA (pH 7.4)), 0.5% SDS, 30% formamide, and 100 µg/l denatured salmon sperm DNA, followed by washing with 1x SSC, 0.1 % SDS solution at 50°C. Even lower stringency conditions include hybridization under the above low stringency condition, with the exception that a solution with high salt concentration (for example, 5x SSC) is used, followed by washing.

The various conditions described above can be also set by adding or changing a blocking reagent that suppresses the background in hybridization experiments. The addition of a blocking reagent as described above may be accompanied by the alteration of a hybridization condition for conditional compatibility.

DNAs that can hybridize under the stringent conditions described above include DNAs having at least 90% or higher, preferably 95% or higher, more preferably 97% or higher, even more preferably 98% or higher, still more preferably 99% or higher homology to the nucleotide sequence of SEQ ID NO: 2, such homology calculated, for example, based on the above-described parameters using the above-described BLAST, FASTA, or the like.

Specifically, the nucleotide sequences of homolog genes of the *yhbC* gene include the nucleotide sequences of the *yhbC* genes derived from *Erwinia carotovora* (Genbank accession no. BX950851), *Pseudomonas fluorescens* (Genbank accession no. AAAT00000000), and *Xanthomonas capestris* (Genbank accession no. AE008922).

Methods of introducing nucleotide deletions into the genes of the chromosomal DNA of a microorganism include methods that utilize homologous recombination. Conventional methods using homologous recombination include methods that use plasmids for homologous recombination, such plasmids produced, for example, by ligating a mutant gene introduced with a nucleotide deletion to a plasmid DNA carrying a drug resistance gene which cannot replicate by itself in the host cells to which the deletion is to be introduced.

After the plasmid for homologous recombination is introduced into host cells by conventional methods, transformed strains in which the plasmid for homologous recombination has been integrated into the chromosomal DNA through homologous recombination may be selected using drug resistance as an indicator. The obtained transformed strains can be cultured in a medium without the drug for several hours to one day, and then applied onto agar medium containing the drug and agar medium without the drug. Strains that do not grow in the former medium but do grow in the latter medium can then be selected to obtain strains whose chromosomal DNAs have undergone a second homologous recombination. Whether a nucleotide deletion has been introduced into a gene of interest in the chromosomal DNA can be confirmed by determining the nucleotide sequence of the region in the chromosomal DNA in which the gene introduced with the nucleotide deletion is present.

Microorganisms to which a nucleotide deletion can be introduced into a gene of interest within their chromosomal DNAs by the methods described above include, for example, microorganisms belonging to the genus *Escherichia.*

Furthermore, methods for efficiently introducing nucleotide deletions into multiple genes, via homologous recombination, include methods that use linear DNAs.

Specifically, in such methods, cells are allowed to incorporate a linear DNA that contains regions present at both outer sides of the target region of the chromosomal DNA into which a nucleotide deletion is to be introduced, and allowed to undergo homologous recombination between the chromosomal DNA and the introduced linear DNA. Such methods are applicable to any microorganism, so long as they efficiently incorporate linear DNAs. Such microorganisms preferably include those belonging to the genus *Escherichia*, more preferably *Escherichia coli,* even more preferably *Escherichia coli* expressing a group of λ phage-derived recombination proteins (the λ Red recombination system).

Examples of *Escherichia coli* that express the λ Red recombination system include the *Escherichia coli* JM101 strain having pKD46 [available from the *Escherichia coli* Genetic Stock Center (Yale University, U.S.A)], which is a plasmid DNA carrying the λ Red recombination system gene.

DNAs used in homologous recombination may include:
(a) linear DNA comprising DNAs present at both outer sides of the target region of the chromosomal DNA into which a nucleotide deletion is to be introduced or DNAs having homology to the DNAs arranged at both ends of a drug resistance gene;
(b) linear DNA in which DNAs present at both outer sides of the target region of the chromosomal DNA into which a nucleotide deletion is to be introduced or DNAs having homology to the DNAs are directly linked together;
(c) linear DNA comprising DNAs present at both outer sides of the target region of the chromosomal DNA into which a nucleotide deletion is to be introduced or DNAs having homology to the DNAs arranged at both ends of a DNA comprising a drug resistance gene and a gene that can be used for negative selection; and
(d) the linear DNA described above in (a), which further comprises a DNA comprising a nucleotide sequence recognized by yeast-derived Flp recombinase [Proc. Natl. Acad. Sci. USA., 82, 5875 (1985)] between the drug resistance gene and the DNAs present at both outer sides of the target region of the chromosomal DNA into which a nucleotide deletion is to be introduced or DNAs having homology to the DNAs.

Any drug resistance gene can be used, so long as it can confer resistance to a drug to which the host microorganism is susceptible. When *Escherichia coli* is used as a host microorganism, examples of drug resistance genes include the kanamycin resistance gene, chloramphenicol resistance gene, gentamicin resistance gene, spectinomycin resistance gene, tetracycline resistance gene, and ampicillin resistance gene.

Genes that can be used for negative selection include genes that are lethal to a host microorganism under specific culture conditions when the genes are expressed in the microorganism. Examples of such genes include the *sacB* gene [Appl. Environ. Microbiol., 59, 1361-1366 (1993)] derived from microorganisms belonging to the genus *Bacillus* and the *rpsL* gene [Genomics, 72, 99-104 (2001)] derived from microorganisms belonging to the genus *Escherichia.*

The above-mentioned DNAs present at both ends of the linear DNA, which are DNAs present outside of both ends of a target region of a chromosomal DNA into which a nucleotide deletion is to be introduced, or DNAs having homology to such DNAs, may be arranged in the linear DNA in the same direction as that of the chromosomal DNA. Their length is preferably about 10 bp to 100 bp, more preferably about 20 bp to 50 bp, and even more preferably about 30 bp to 40 bp.

The nucleotide sequence recognized by yeast-derived Flp recombinase is not particularly limited, so long as the protein recognizes the nucleotide sequence and catalyzes homologous recombination. Such a nucleotide sequence preferably includes a DNA comprising the nucleotide sequence of SEQ ID NO: 3 as well as DNAs comprising the nucleotide sequence in which one to several nucleotides in the nucleotide sequence of SEQ ID NO: 3 are deleted, substituted, or added, and the resulting DNA is recognized by yeast-derived Flp recombinase and whose homologous recombination is catalyzed by the recombinase.

The phrase "having homology" means that the above-described linear DNA has homology that allows homologous recombination in a region of interest in the chromosomal DNA. Specifically, such homology include 80% or higher, preferably 90% or higher, more preferably 95% or higher, and even more preferably 100% homology.

The above-described nucleotide sequence homology can be determined using programs such as BLAST and FASTA as described above.

The above-described linear DNA can be produced by PCR. Alternatively, linear DNAs of interest can also be obtained by constructing a DNA including the above-described linear DNA with a plasmid and treating it with restriction enzymes.

Methods for introducing a nucleotide deletion, substitution, or addition into the chromosomal DNA of a microorganism include the following methods 1 to 4.

### Method 1:

A method in which the linear DNA described above in (a) or (d) is introduced into host microorganisms to yield transformed strains in which the linear DNA has been inserted into the chromosomal DNA through homologous recombination that may be selected using drug resistance as an indicator.

### Method 2:

A method in which the linear DNA described above in (b) is introduced into transformed strains obtained by the method 1 described above and a region in the chromosomal DNA of the microorganism is substituted or deleted by removing the drug gene inserted into the chromosomal DNA by the method.

### Method 3:

A method in which:
[1] the linear DNA described above in (c) is introduced into host microorganisms and transformed strains in which the linear DNA has been inserted into the chromosomal DNA through homologous recombination are selected using drug resistance as an indicator;
[2] DNAs in which DNAs having homology to DNAs positioned outside of both ends of a region targeted for a nucleotide substitution or deletion in the chromosomal DNA are arranged in the same order as in the chromosomal DNA are synthesized, and introduced into the transformed strains obtained above in [1]; and
[3] the transformed strains treated as described above in [2] are cultured under a condition in which a gene applicable to negative selection is expressed, and strains that can grow in the culture are selected as strains in which the drug resistance gene and the gene applicable to negative selection have been removed from the chromosomal DNA.

### Method 4:

A method in which:
[1] the linear DNA described above in (d) is introduced into host microorganisms and transformed strains in which the linear DNA has been inserted into the chromosomal DNA through homologous recombination are selected using drug resistance as an indicator; and
[2] an expression plasmid for Flp recombinase gene is introduced into transformed strains obtained above in [1] to express the gene, and then strains sensitive to the drug used in [1] above are obtained.

Any method for introducing linear DNAs into host microorganisms may be used in the methods described above, so long as the method allows for the introduction of DNA into microorganisms. Such methods include, for example, methods using calcium ion [Proc. Natl. Acad. Sci., USA, 69, 2110 (1972)], protoplast method (JP-A (Kokai) S63-2483942), and electroporation method [Nucleic Acids Res., 16, 6127 (1988)].

An arbitrary gene can be inserted into the chromosomal DNA simultaneously upon removal of the drug resistance gene and the like using the linear DNA of Method 2 or Method 3 [2] in which an arbitrary gene to be inserted into the chromosomal DNA is integrated near the center of the linear DNA.

In Methods 2 to 4 described above, foreign genes, such as the drug resistance gene and a gene applicable to negative selection, will not remain in the chromosomal DNAs of the finally obtained transformed strains. Therefore, by using the methods, microorganisms having nucleotide deletions or the like in two or more regions at different positions in the chromosomal DNA can be readily produced by repeating the procedure of the methods using the same drug resistance gene and gene applicable to negative selection.

### 3. Process for producing useful substances of the present invention:

Useful substances can be produced by culturing in a medium the microorganisms that are used in the production process of the present invention described above in section 2, allowing for the production and accumulation of the useful substance in the culture, and recovering the useful substance from the culture.

Methods of culturing microorganisms in the medium can be performed according to conventional methods for culturing microorganisms.

Specifically, both natural media and synthetic media may be used, so long as they contain a carbon source that can be assimilated by the microorganisms, a nitrogen source, inorganic salts, and the like and allow for the efficient culture of the microorganisms.

Any carbon sources may be used, so long as they can be assimilated by the microorganisms; examples include glucose, fructose, sucrose, molasses containing them, carbohydrates such as starch and starch hydrolysates, organic acids such as acetic acid and propionic acid, and alcohols such as ethanol and propanol.

As nitrogen sources, ammonia; ammonium chloride; ammonium salts of inorganic and organic acids such as ammonium sulfate, ammonium acetate, and ammonium phosphate; other nitrogen-containing compounds; peptone, meat extract, yeast extract, corn steep liquor, casein hydrolysate, soybean cake, soybean cake hydrolysate, and various bacterial cells from fermentation broth; digests thereof; and the like can be used.

As inorganic salts, monopotassium phosphate, dipotassium phosphate, magnesium phosphate, magnesium sulfate, sodium chloride, ferrous sulfate, manganese sulfate, copper sulfate, calcium carbonate, and the like can be used.

Generally, culture is achieved under aerobic conditions, including the steps of shaking culture or stirring culture with aeration. Preferred culture temperatures range from 15 to 40°C. In general, the culture period is five hours to seven days. The pH is kept at 3.0 to 9.0 during culture. The pH may be adjusted using an inorganic or organic acid, alkaline solution, urea, calcium carbonate, ammonia, and the like.

If necessary, antibiotics, such as ampicillin and tetracycline, may be added to the medium during culture.

When microorganisms transformed with an expression vector having an inducible promoter are cultured, an inducer may be added to the medium if needed. For example, when microorganisms transformed with an expression vector having the lac promoter are cultured, isopropyl-β-D-thiogalactopyranoside or the like may be added to the medium; and when microorganisms transformed with an expression vector having the trp promoter are cultured, indoleacrylic acid or the like may be added to the medium.

Useful substances produced and accumulated in a culture can be collected according to conventional methods, using for example activated carbon or ion exchange resins, or by extraction using organic solvents, crystallization, thin layer chromatography, high performance liquid chromatography, or the like.

Examples are described below; however, the present invention should not be construed as being limited thereto. Methods described in "Molecular Cloning, A Laboratory Manual, Third Edition, Cold Spring Harbor Laboratory Press (2001)" can be appropriately used for DNA manipulation or the like described in the Examples below.

### [Example 1] Preparation of strains lacking the yhbC gene

Using the method described below, a stock of P1 phage was prepared from *E. coli* BW25113Δ*yhbC::km* lacking the *yhbC* gene (hereinafter referred to as *E. coli* ΔyhbC strain) obtained from the Nara Institute of Science and Technology.

*E. coli* ΔyhbC strain was cultured using liquid LB medium [which contains 10 g/l Bacto tryptone (Difco), 5 g/l Bacto yeast extract (Difco), and 5 g/l of sodium chloride, and to which 1 mol/l sodium hydroxide was added at a proportion of 1 ml/1] containing 20 mg/l kanamycin at 30°C overnight. Then, 0.5 ml of the culture was combined and mixed with 1.4 ml of fresh liquid LB medium and 100 µl of 0.1 mol/l calcium chloride solution.

After the mixed solution was cultured with shaking at 30°C for five to six hours, 400 µl of the mixture was transferred to a sterile tube. 2 µl of the P1 phage stock solution was added thereto. After ten minutes of incubation at 37°C, 3 ml of soft agar solution (which contains 10g/l Bacto tryptone, 5 g/l Bacto yeast extract, 5 mmol/l calcium chloride, 3 g/l agar, and to which 1 mol/l sodium hydroxide was added at a proportion of 1 ml/1) incubated at 50°C was added, and the mixture was stirred well. The mixture was then plated onto Ca-LB agar plates (which contains 10 g/l Bacto tryptone, 5 g/l Bacto yeast extract, 5 mmol/l calcium chloride, 15 g/l agar, and to which 1 mol/l sodium hydroxide was added at a proportion of 1 ml/1; the diameter of the plate was 15 cm).

After the plate was incubated at 37°C for seven hours, soft agar portions were broken into fine pieces with a spreader and collected. The pieces were centrifuged at 1,500 x g and 4°C for ten minutes. 100 µl of chloroform was added to 1.5 ml of the supernatant, and the mixture was stored at 4°C overnight. On the next day, the liquid was centrifuged at 15,000 x g for two minutes, and the supernatant was stored as a phage stock at 4°C.

Then, *E. coli* W3110 and *E. coli* MG1655 strains were transformed with the obtained phages. *E. coli* W3110 and *E. coli* MG1655 strains were cultured in liquid Ca-LB medium at 30°C for four hours, and a 200-µl aliquots of the cultures were taken out, and 1 µl of the phage stock was added. After ten minutes of incubation at 37°C, 5 ml of liquid LB medium and 200 µl of 1 mol/l sodium citrate solution were added, and the resulting mixture was stirred.

After ten minutes of centrifugation at 1,500 x g and 25°C, the supernatant was discarded. 1 ml of liquid LB medium and 10 µl of 1 mol/l sodium citrate solution were added to the precipitated cells. The mixture was incubated at 30°C for two hours. 100 µl of the solution was applied onto antibiotic medium 3 agar plates (0.15% meat extract, 0.15% yeast extract, 0.5% peptone, 0.1% glucose, 0.35% sodium chloride, 0.132% dipotassium hydrogen phosphate (dipotassium phosphate), and 1.5% agar (pH 7.0)) containing 20 mg/l kanamycin. The plates were incubated at 30°C overnight.

Twenty four colonies formed from each strain introduced with a deletion were independently applied onto antibiotic medium 3 agar plates containing 20 mg/l kanamycin to confirm their drug sensitivity. *E. coli* W3110 and *E. coli* MG1655 strains to which the deletion of the *yhbC* gene [*ΔyhbC::km*] had been transduced were obtained by selecting kanamycin resistant strains (hereinafter, referred to as *E. coli* W3110ΔyhbC strain and *E. coli* MG1655ΔyhbC strain, respectively).

### [Example 2] Analysis of the growth characteristics of strains lacking yhbC

Each of *E. coli* BW25113ΔyhbC strain, *E. coli* W3110ΔyhbCstrain, and *E. coli* MG1655ΔyhbC strain, and the control strains, *E. coli* BW25113 strain, *E. coli* W3110 strain, and *E. coli* MG1655 strain, was inoculated to a test tube containing 5 ml of liquid LB medium. The strains were cultured with shaking at 30°C for 20 hours to obtain the cultures. 50 µl of the cultures were inoculated to L-shaped test tubes containing 5 ml of liquid M9 medium (1% glucose, 0.6% disodium hydrogen phosphate, 0.3% potassium dihydrogen phosphate (monopotassium phosphate), 0.05% sodium chloride, 0.1 % ammonium chloride, 0.012% magnesium sulfate, 0.0015% calcium chloride, and 0.0011% ferrous sulfate). The bacteria were cultured with shaking at 30°C. The turbidity (absorbance at 660 nm; hereinafter described as OD660nm) was measured every ten minutes after the start of culture. The period where OD660nm value is within the range of 0.3 to 1.0 was confirmed to be the logarithmic growth phase. The specific growth rate µ [=Δ In(OD660nm)/ Δ culture period] for the period was calculated.

µ values of *E. coli* BW25113ΔyhbC, *E. coli* W3110ΔyhbC, and *E. coli* MG1655ΔyhbC strains, which are strains lacking the *yhbC* gene, were 0.34, 0.34, and 0.35, respectively, while µ values of their parental strains, *E. coli* BW25113, *E. coli* W3110, and *E. coli* MG1655 strains, were 0.31, 0.28, and 0.28, respectively. Specifically, all strains lacking the *yhbC* gene exhibited greatly improved growth characteristic in liquid minimal M9 medium as compared to the parental strains.

The results described above demonstrate that useful substances can be efficiently produced by using microorganisms that lack the *yhbC* gene.

### [Example 3] Preparation of xylitol-producing strains

The ability to produce xylitol was conferred to *E. coli* BW25113 strain by the method described below.

### (1) Preparation of E. coli W3110red strain capable of homologous recombination with a linear DNA on its chromosomal DNA

By using the same method as described in Example 1, P1 phages were prepared from *E. coli* KM22 strain [Gene, 246, 321-330 (2000)] capable of homologous recombination between a linear DNA and a chromosomal DNA.

Then, *E. coli* W3110 strain was transformed with the obtained phage. *E. coli* W3110 strain was cultured in liquid Ca-LB medium at 30°C for four hours. A 200-µl aliquot of the cultures was taken out, and 1 µl of the phage stock was added. After ten minutes of incubation at 37°C, 5 ml of liquid LB medium and 200 µl of 1 mol/l sodium citrate solution were added and the resulting mixture was stirred.

After ten minutes of centrifugation at 1,500 x g and 25°C, the supernatant was discarded. 1 ml of liquid LB medium and 10 µl of 1 mol/l sodium citrate solution were added to the precipitated cells. The mixture was incubated at 30°C for two hours. 100 µl of the solution was applied onto antibiotic medium 3 agar plates containing 20 mg/l kanamycin. The plates were incubated at 30°C overnight. Twenty four colonies formed from each strain introduced with a deletion were independently applied onto antibiotic medium 3 agar plates containing 20 mg/l kanamycin to confirm their drug sensitivity. W3110red strain transduced with the chromosomal DNA region [Δ(recC ptr recB recD)::Plac-red kan] capable of homologous recombination between a linear DNA and chromosomal DNA was obtained by selecting kanamycin resistant strains.

### (2) Construction of strains introduced with a group of genes for xylitol production

### (i) Isolation of xylose reductase gene

A 1-kbp DNA fragment containing the xylose reductase gene *(XYL1)* was amplified by PCR using *Kluyveromyces lactis* chromosomal DNA as a template and a primer set of DNAs each consisting of the nucleotide sequence of SEQ ID NO: 4 or 5. 25 µl of a reaction solution containing 10 ng of chromosomal DNA and 20 pmol each of the primer DNAs was prepared according to the instructions appended to LA-Taq (TaKaRa Bio). PCR was carried out using LA-Taq under the following conditions: incubation at 94°C for two minutes, followed by 30 cycles of 94°C for 30 seconds, 55°C for 30 seconds, and 72°C for one minute, and subsequent incubation at 72°C for ten minutes.

After the amplification of a DNA fragment of interest was confirmed by agarose gel electrophoresis, the DNA fragment was excised and purified from the gel. The plasmid pDrive-XYL1 was obtained by cloning the fragment into the plasmid pDrive (QIAGEN) using the PCR Cloning Kit (QIAGEN).

### (ii) Introduction of xylose permease gene

A 1.5-kbp DNA fragment containing the Shine-Dalgarno sequence and xylose permease gene *(xylE)* was amplified by PCR using *E. coli* chromosomal DNA as a template and a primer set of DNAs each consisting of the nucleotide sequence of SEQ ID NO: 6 or 7. 25 µl of a reaction solution containing 10 ng of chromosomal DNA and 20 pmol each of the primer DNAs was prepared according to the instructions appended to LA-Taq. PCR was carried out using LA-Taq under the following conditions: incubation at 94°C for two minutes, followed by 30 cycles of 94°C for 30 seconds, 55°C for 30 seconds, and 72°C for 1.5 minutes, and subsequent incubation at 72°C for ten minutes.

After the amplification of a DNA fragment of interest was confirmed by agarose gel electrophoresis, the DNA fragment was excised and purified from the gel. The plasmid pDrive-xylE was obtained by cloning the fragment into the plasmid pDrive using the PCR Cloning Kit.

### (iii) Isolation of the tetracycline resistance gene

A 1.5-kbp DNA fragment containing the tetracycline resistance gene *(tet)* was amplified by PCR using as a template the chromosomal DNA of *E. coli* CGSC7465 strain carrying Tn10 transposon in its chromosomal DNA (available from the Coli Genetic Stock Center managed by the Yale university in the U. S. A) and a primer set of DNAs each consisting of the nucleotide sequence of SEQ ID NO: 8 or 9.

25 µl of a reaction solution containing 10 ng of chromosomal DNA and 20 pmol each of the primer DNAs was prepared according to the instructions appended to LA-Taq. PCR was carried out using LA-Taq under the following conditions: incubation at 94°C for two minutes, followed by 30 cycles of 94°C for 30 seconds, 55°C for 30 seconds, and 72°C for 1.5 minutes, and subsequent incubation at 72°C for ten minutes.

After the amplification of a DNA fragment of interest was confirmed by agarose gel electrophoresis, the DNA fragment was excised and purified from the gel. The plasmid pDrive-tet was obtained by cloning the fragment into the plasmid pDrive using the PCR Cloning Kit.

### (iv) Construction of pQE60-XYLl-xylE-tet plasmid

After the plasmid pDrive-XYL1 was cleaved using the restriction enzymes *Nco*I and *Bam*HI, a DNA fragment containing *XYL1* was separated by agarose gel electrophoresis, and collected and purified from the gel using the QIA quick Gel Extraction Kit (QIAGEN). Likewise, the plasmid pQE60 (QIAGEN) was cleaved using the restriction enzymes *Bsp*HI and *Bam*HI, and then purified using QIA quick PCR Purification Kit (QIAGEN). The DNA fragment containing *XYL1* was ligated with the DNA fragment of pQE60 using the DNA ligation kit ver. 2 (TaKaRa Bio) according to the appended instructions.

10 µl of ligation solution was mixed with 100 µl of *E. coli* JM109 competent cells (TaKaRa Bio). The resulting mixture was incubated on ice for 20 minutes and then at 42°C for 30 seconds, and then incubated on ice for two minutes. 1 ml of liquid SOC medium was added to the mixture, and a 100-µl aliquot was applied onto an LB agar medium containing 20 mg/l ampicillin, and then incubated at 30°C overnight. The plasmid pQE60-XYL1 containing *XYL1* was obtained from the formed colony.

Next, the plasmid pDrive-xylE was cleaved using the restriction enzymes *Bam*HI and *Bgl*II. Then, a DNA fragment containing *xylE* was separated by agarose gel electrophoresis, and collected and purified from the gel using the QIA quick Gel Extraction Kit. Likewise, the above-mentioned plasmid pQE60 XYL1 was cleaved using the restriction enzyme *Bam*HI, and then purified using the QIA quick PCR Purification Kit. The DNA fragment containing *xylE* was ligated with the DNA fragment of pQE60 XYL1 using the DNA ligation kit ver. 2 according to the appended instructions. Then, the plasmid pQE60-XYL1-xylE containing both *XYL1* and *xylE* was obtained by the same procedure as described in (i).

Next, the plasmid pDrive-tet was cleaved using the restriction enzyme *Nhe*I*.* Then, a DNA fragment containing *tet* was separated by agarose gel electrophoresis, and collected and purified from the gel using QIA quick Gel Extraction Kit. Likewise, the above-mentioned plasmid pQE60-XYL1-xy1E was cleaved using the restriction enzyme *Nhe*I, and then purified using the QIA quick PCR Purification Kit. The DNA fragment containing *tet* was ligated with the DNA fragment of pQE60-XYL1-xy1E using the DNA ligation kit ver. 2 according to the appended instructions. Then, the plasmid pQE60-XYL1-xy1E-tet containing all of *XYL1*, *xylE*, and *tet* was obtained by the same procedure as described in (i).

### (v) Construction of E. coli W3110redΔxylA::XYL1 xylE tet strain

A DNA fragment containing the xylose reductase gene, xylose permease gene, and tetracycline resistance gene arranged in the center and a DNA having the nucleotide sequence of SEQ ID NO: 12 [DNA at the 5' end of the xylose isomerase gene (*xylA*)] and a DNA having the nucleotide sequence shown in SEQ ID NO: 13 [DNA at the 3' end of the xylose isomerase gene (*xylA*)] positioned at its ends was amplified by PCR using the above-described pQE60-XYL1-xylE-tet plasmid as a template and a primer set of DNAs each consisting of the nucleotide sequence of SEQ ID NO: 10 or 11.

25 µl of a reaction solution containing 10 ng of plasmid DNA and 20 pmol each of primer DNAs was prepared according to the instructions appended to LA-Taq. PCR was carried out using LA-Taq under the following conditions: incubation at 94°C for two minutes, followed by 30 cycles of 94°C for 30 seconds, 55°C for 30 seconds, and 72°C for five minutes, and subsequent incubation at 72°C for ten minutes.

Then, *E. coli* W3110red strain was transformed with 1 µg of the DNA. Transformation was carried out by the same condition of electroporation as described above in (1).

After the transformed cells were cultured using 1 ml of liquid SOC medium containing 2 mmol/l isopropyl-β-D-thiogalactoside at 30°C for three hours, they were applied onto an LB agar plate containing 50 µg/ml chloramphenicol (liquid LB medium containing 1.5% agar; hereinafter abbreviated as LBcm agar plate) and incubated at 37°C overnight.

The grown *E. coli* W3110red strain was confirmed to be a strain in which the xylA gene had been replaced with the xylose reductase gene, xylose permease gene, and tetracycline resistance gene in its chromosomal DNA, and named as *E*. *coli* W3110redΔ*xylA*::*XYL1 xylE tet* strain.

### (vi) Construction of E. coli BW25113 ΔxylA::XYL1 xylE tet and E. coli BW25113 ΔyhbC::km ΔxylA::XYL1 xylE tet strains

P1 phage was prepared from *E. coli* W3110redΔ*xylA::XYL1 xylE tet* strain by the same method as described in Example 1.

Next, *E. coli* BW25113 and *E. coli* BW25113 Δ*yhbC*::*km* strains were transformed using the obtained phage. *E. coli* BW25113 and *E. coli* BW25113 *ΔyhbC::km* strains were cultured using liquid Ca-LB medium at 30°C for four hours. A 200-µl aliquot of the cultures was taken out and 1 µl of P1 phage stock obtained from *E. coli* W3110redΔ*xylA*::*XYL1 xylE tet* strain was added. After ten minutes of incubation at 37°C, 5 ml of liquid LB medium and 200 µl of 1 mol/l sodium citrate solution were added. The mixtures were stirred.

After ten minutes of centrifugation at 1,500 x g and 25°C, the supernatant was discarded. 1 ml of liquid LB medium and 10 µl of 1 mol/l sodium citrate solution were added to the precipitated cells. The mixture was incubated at 30°C for two hours. 100 µl of the solution was applied onto antibiotic medium 3 agar plates containing 20 mg/l kanamycin. The plates were incubated at 30°C overnight. Twenty four colonies formed from each strain introduced with a deletion were independently applied onto antibiotic medium 3 agar plates containing 10 mg/l tetracycline to confirm their drug sensitivity. The transduced strains, *E. coli* BW25113 Δ*xylA*::*XYL1 xylE tet* and *E. coli* BW25113 Δ*yhbC::km* Δ*xylA*::*XYL1 xylE tet,* in which the xylA gene had been replaced with a DNA region containing the xylose reductase gene, xylose permease gene, and tetracycline resistance gene in the chromosomal DNA, were obtained by selecting tetracycline resistant strains.

### [Example 4] Production of useful substances using strains lacking the yhbC gene (1)

Each of *E. coli* BW25113 *ΔxylA::XYL1 xylE tet* and *E. coli* BW25113 *ΔyhbC::km* Δ*xylA*::*XYL1 xylE tet* strains was inoculated to a test tube containing 5 ml of liquid LB medium, and cultured with shaking at 30°C for 20 hours to obtain the cultures. 30 ml of the cultures were inoculated to a 2,000-ml jar fermenter containing 1,000 ml of liquid MX medium (3% glucose, 6% xylose, 0.0024% isopropyl-β-D-thiogalactoside, 0.6% disodium hydrogen phosphate, 0.3% potassium dihydrogen phosphate, 0.05% sodium chloride, 0.1% ammonium chloride, 0.012% magnesium sulfate, 0.0015% calcium chloride, and 0.0011 % ferrous sulfate), and cultured at 30°C. In the culture described above, added xylose was converted into xylitol in a stoichiometric manner.

Portions of the cultures were sampled 23, 28.5, and 32.5 hours after the start of culture. The samples were centrifuged and the supernatants were diluted 1,000 times. The amount of xylitol accumulated in the culture liquid was measured using Ion Chromatograph System DXa-500 of DIONEX. The growth was quantified by measuring the turbidity of culture (OD660nm) using a spectrophotometer. The result is shown in Table 1.

**[Table 1]**

| Bacterial strain | Quantitation item | Culture period (hour) | | |
|---|---|---|---|---|
| | | 23 | 28.5 | 32.5 |
| BW25113Δ*xylA*::*XYL1 xylE tet* strain | Xylitol (g/l) | 2.7 | 6.0 | 9.2 |
| | OD660nm | 3.9 | 7.8 | 11.3 |
| BW25113Δ*yhbC*::*km* Δ*xylA*::*XYL1 xylE tet* strain | Xylitol (g/l) | 5.1 | 11.4 | 16.0 |
| | OD660nm | 6.6 | 10.7 | 12.2 |

### [Example 5] Production of useful substances using strains that lack the yhbC gene (2)

*E. coli* W3110Δ*yhbC::km ΔxylA*::*XYL1 xylE tet* strain was obtained by transforming *E. coli* W3110ΔyhbC strain using P1 phage prepared from *E. coli* W3110redΔ*xylA*::*XYL1 xylE tet* strain by the same method as described in Example 3. *E. coli* W3110 Δ*xylA*::*XYL1 xylE tet* strain was obtained by transforming *E. coli* W3110 using the same P1 phage.

*E. coli* W3110Δ*xylA*::*XYL1* xylE tet and *E. coli* W3110Δ*yhbC::km* Δ*xylA*::*XYL1 xylE tet* strains were independently inoculated to test tubes containing 5 ml of liquid LB medium containing 10 g/l glucose, and cultured with shaking at 30°C for 16 hours. Thus, the cultures were obtained. 3.75 ml of the cultures were inoculated to 250-ml jar fermenter containing 125 ml of a liquid medium (60 g/l glucose, 10 g/l yeast extract, 6.8 g/l disodium hydrogen phosphate, 3 g/l potassium dihydrogen phosphate, 0.5 g/l sodium chloride, 1 g/l ammonium chloride, 0.247 g/l magnesium sulfate, 0.015 g/l calcium chloride, and 0.028 g/l ferrous sulfate). The culture was started at 30°C and pH 7.0 (adjusted with ammonia water) with an aeration volume of 1 vvm. Stirring was started at 500 rpm, and the stirring rate was changed to keep the concentration of dissolved oxygen at 1 ppm. 3.5 hours after the start of culture, xylose was added to become 80 g/l, and the culture was continued.

In the culture described above, added xylose was converted into xylitol in a stoichiometric manner.

Portions of the cultures were sampled 23.3, 29.3, 33.3 and 47.3 hours after the start of culture. The samples were centrifuged and the concentration of xylitol in the culture and the turbidity of the culture were measured by the same methods described in Example 4. The result is shown in Table 2.

**[Table 2]**

| Bacterial strain | Quantitation item | Culture period (hour) | | | |
|---|---|---|---|---|---|
| | | 23.3 | 29.3 | 33.3 | 47.3 |
| W311Δ*xylA*::*XYL1 xylE tet* strain | Xylitol (g/l) | 22.1 | 34.7 | 39.3 | 57.2 |
| | OD660nm | 15.2 | 16.8 | 16.5 | 16.3 |
| W3110Δ*yhbC::km* Δ*xylA*::*XYL1 xylE tet* strain | Xylitol (g/l) | 28.9 | 42.1 | 48.8 | 66.4 |
| | OD660nm | 16.6 | 17.2 | 17.0 | 17.8 |

As shown in Tables 1 and 2, it was revealed that the growth rate and xylitol productivity is improved in the strain that lacks the *yhbC* gene and that the degree of productivity improvement is greater than that of the growth.

The results described above demonstrates that the improvement of growth characteristics in the strain lacking the *yhbC* gene not only leads to an improvement in the productivity of useful substances, specifically various useful substances produced in the fermentation process, for example, proteins, nucleic acids, vitamins, sugars, organic acids, and lipids but also leads to an improvement in productivity that is greater than the degree of the improvement of growth characteristics.

### Industrial Applicability

Useful substances can be efficiently produced according to the present invention.

### Sequence Listing Free Text

SEQ ID NO: 3 - Note for the artificial sequence; synthetic DNA
SEQ ID NO: 4 - Note for the artificial sequence; synthetic DNA
SEQ ID NO: 5 - Note for the artificial sequence; synthetic DNA
SEQ ID NO: 6 - Note for the artificial sequence; synthetic DNA
SEQ ID NO: 7 - Note for the artificial sequence; synthetic DNA
SEQ ID NO: 8 - Note for the artificial sequence; synthetic DNA
SEQ ID NO: 9 - Note for the artificial sequence; synthetic DNA
SEQ ID NO: 10 - Note for the artificial sequence; synthetic DNA
SEQ ID NO: 11 - Note for the artificial sequence; synthetic DNA
SEQ ID NO: 12 - Note for the artificial sequence; synthetic DNA
SEQ ID NO: 13 - Note for the artificial sequence; synthetic DNA

## Claims

1. A process for producing a useful substance, which comprises culturing a microorganism that lacks from its chromosomal DNA all or part of a gene encoding a protein comprising the amino acid sequence of SEQ ID NO: 1 or a gene encoding a protein that has 80% or more homology to the amino acid sequence of SEQ ID NO: 1 in a medium so as to produce and accumulate the useful substance in the culture, and recovering the useful substance from the culture.

2. The process according to claim 1, wherein the gene encoding a protein comprising the amino acid sequence of SEQ ID NO: 1 has the nucleotide sequence of SEQ ID NO: 2.

3. The process according to claim 1 or 2, wherein the ability of the microorganism to produce the useful substance has been improved by a method selected from the following (1) to (5):
(1) a method of partially or completely releasing at least one of the mechanisms that regulate the biosynthesis of the useful substance;
(2) a method of enhancing expression of at least one of the enzymes involved in the biosynthesis of the useful substance;
(3) a method of increasing the copy number of at least one of the enzyme genes involved in the biosynthesis of the useful substance;
(4) a method of partially or completely blocking at least one of the branched metabolic pathways of the biosynthetic pathway of the useful substance to metabolites other than the useful substance; and
(5) a method of selecting a cell strain that is more resistant to an analog of the useful substance as compared with a wild-type strain.

4. The process according to any one of claims 1 to 3, wherein the microorganism belongs to the genus *Escherichia, Erwinia, Pseudomonas, Rhodobacter, Salmonella, Serratia, Vibrio*, *Xanthomonas,* or *Xylella.*

5. The process according to any one of claims 1 to 4, wherein the microorganism is a microorganism of the species *Escherichia coli, Erwinia carotovora, Pseudomonas putida, Pseudomonas fluorescens, Pseudomonas aeruginosa, Rhodobacter capsulatus, Salmonella typhimurium, Serratia marcescens, Vibrio fischeri, Xanthomonas capestris,* or *Xylella fastidiosa.*

6. The process according to any one of claims 1 to 5, wherein the useful substance is selected from the group consisting of a protein, peptide, amino acid, nucleic acid, vitamin, sugar, sugar alcohol, alcohol, organic acid, and lipid.


Sequence listing

<110> KYOWA HAKKO KOGYO CO., LTD.

<120> Process for producing useful substance

<130> K 10008 EP

<150> 2005-09-29
<151> JP2005-284539

<160> 13

<170> PatentIn Ver. 2.1

<210> 1
<211> 152
<212> PRT
<213> Escherichia coli

<400> 1
Met Gly Leu Ser Thr Leu Glu Gln Lys Leu Thr Glu Met Ile Thr Ala
1               5                   10                  15

Pro Val Glu Ala Leu Gly Phe Glu Leu Val Gly Ile Glu Phe Ile Arg
            20                  25                  30

Gly Arg Thr Ser Thr Leu Arg Ile Tyr Ile Asp Ser Glu Asp Gly Ile
        35                  40                  45

Asn Val Asp Asp Cys Ala Asp Val Ser His Gln Val Ser Ala Val Leu
    50                  55                  60

Asp Val Glu Asp Pro Ile Thr Val Ala Tyr Asn Leu Glu Val Ser Ser
65                  70                  75                  80

Pro Gly Leu Asp Arg Pro Leu Phe Thr Ala Glu His Tyr Ala Arg Phe
                85                  90                  95

Val Gly Glu Glu Val Thr Leu Val Leu Arg Met Ala Val Gln Asn Arg
            100                 105                 110

Arg Lys Trp Gln Gly Val Ile Lys Ala Val Asp Gly Glu Met Ile Thr
        115                 120                 125

Val Thr Val Glu Gly Lys Asp Glu Val Phe Ala Leu Ser Asn Ile Gln
    130                 135                 140

Lys Ala Asn Leu Val Pro His Phe
145                 150


<210> 2
<211> 832
<212> DNA
<213> Escherichia coli

<220>
<221> CDS
<222> (374)..(829)
<223>

<400> 2

taatgcctgc agattttacg tcccgtctcg gtacaccaaa tcccagcagt atttgcattt 60

tttacccaaa acgagtagaa tttgccacgt ttcaggcgcg gggtggagca gcctggtagc 120

tcgtcgggct cataacccga agatcgtcgg ttcaaatccg gcccccgcaa ccactttccc 180

ttagagtcct ttttcaaata tactgtgaag acttcggcct tcgtagtggg atttgaaaaa 240

atccttctgg aaagtgctcc agaccgcagt tgcggttata gggttcagtt atataaagcc 300

ccgatttatc ggggtttttt gttatctgac tacagaataa ctgggcttta ggcccttttt 360

ttatgtcttg ggg gtg ggc ttg tcc aca tta gag caa aaa tta aca gag 409
Met Gly Leu Ser Thr Leu Glu Gln Lys Leu Thr Glu
1               5                   10

atg att act gcg cca gtt gag gcc ctg ggt ttt gaa ctg gtt ggc atc 457
Met Ile Thr Ala Pro Val Glu Ala Leu Gly Phe Glu Leu Val Gly Ile
        15                  20                  25

gaa ttt att cgc ggt cgc aca tcc aca ctg cgc atc tat att gat agt 505
Glu Phe Ile Arg Gly Arg Thr Ser Thr Leu Arg Ile Tyr Ile Asp Ser
    30                  35                  40

gaa gat ggc atc aat gtt gat gat tgt gct gat gtg agc cac cag gta 553
Glu Asp Gly Ile Asn Val Asp Asp Cys Ala Asp Val Ser His Gln Val
45                  50                  55                  60

agt gct gtg ctg gat gtt gaa gat ccc atc acc gtt gct tat aac ctg 601
Ser Ala Val Leu Asp Val Glu Asp Pro Ile Thr Val Ala Tyr Asn Leu
                65                  70                  75

gaa gtc tcc tca ccg ggt ctc gat cgc cca ctg ttc acg gct gaa cac 649
Glu Val Ser Ser Pro Gly Leu Asp Arg Pro Leu Phe Thr Ala Glu His
            80                  85                  90

tac gcc cgt ttt gtc gga gaa gag gtg act ctg gtt ctc cgt atg gcg 697
Tyr Ala Arg Phe Val Gly Glu Glu Val Thr Leu Val Leu Arg Met Ala
        95                  100                 105

gta caa aac cgt cgt aaa tgg cag ggc gtt atc aaa gcg gta gac ggt 745
Val Gln Asn Arg Arg Lys Trp Gln Gly Val Ile Lys Ala Val Asp Gly
    110                 115                 120

gaa atg atc aca gtt acc gtc gaa ggt aaa gat gaa gtg ttc gcg ctg 793
Glu Met Ile Thr Val Thr Val Glu Gly Lys Asp Glu Val Phe Ala Leu
125                 130                 135                 140

agt aat atc cag aag gcg aac ctg gtt ccc cac ttt taa 832
Ser Asn Ile Gln Lys Ala Asn Leu Val Pro His Phe
                145                 150

<210> 3
<211> 34
<212> DNA
<213> Artificial Sequence

<400> 3
gaagttccta ttctctagaa agtataggaa cttc 34

<210> 4

<211> 32
<212> DNA
<213> Artificial Sequence

<400> 4
tcatgacgta cttagcagaa acagttactt ta                                    32


<210> 5
<211> 32
<212> DNA
<213> Artificial Sequence

<400> 5
ggatccttag atgaaagttg ggaattcgtt gt                                    32


<210> 6
<211> 57
<212> DNA
<213> Artificial Sequence

<400> 6
ggatccatta aagaggagaa attaaccatg aatacccagt ataattccag ttatata    57


<210> 7
<211> 36
<212> DNA
<213> Artificial Sequence

<400> 7
agatctttac agcgtagcag tttgttgtgt tttctt                                36


<210> 8
<211> 26
<212> DNA
<213> Artificial Sequence

<400> 8
gctagcagct ctaatgcgct gttaat                                           26


<210> 9
<211> 26
<212> DNA
<213> Artificial Sequence

<400> 9
gctagcaggc caatttattg ctattt                                           26


<210> 10
<211> 65
<212> DNA
<213> Artificial Sequence

<400> 10
cgctaccgat aaccgggcca acggactgca cagttagccg tccattttag cttccttagc 60

tcctg                                                                  65

<210> 11
<211> 65
<212> DNA
<213> Artificial Sequence

<400> 11
catccatcac ccgcggcatt acctgattat ggagttcaat ctcgagaaat cataaaaaat 60

ttatt 65


<210> 12
<211> 40
<212> DNA
<213> Artificial Sequence

<400> 12
catccatcac ccgcggcatt acctgattat ggagttcaat 40


<210> 13
<211> 40
<212> DNA
<213> Artificial Sequence

<400> 13
cgctaccgat aaccgggcca acggactgca cagttagccg 40